# EUROPEAN PATENT APPLICATION

(11) **EP 1 906 329 A1**
(43) Date of publication of application: **02.04.2008**
(21) Application number: 07123046.0
(22) Date of filing: 07.04.2003
(51) Int. Cl.: G06F 19/00

(54) **Patient monitoring system and method**

(30) Priority: 23.04.2002 US 374902 P; 03.04.2003 US 406376
(62) Divisional of application: 03747272.7
(71) Applicant: Draeger Medical Systems, Inc., Danvers, MA 01923 (US)
(72) Inventor: Manetta, Amy, M., North Billerica, NY 01862 (US); Rutledge, Jolyn, Amesbury, MA 01913 (US); Shaffer, Judith, Orchard Park, NY 14127 (US)
(74) Representative: Kietzmann, Lutz

(57) **Abstract**

The invention relates to a method and system for processing medical information, comprising the activities of storing information including sets of patient medical data and chart setups associated with a plurality of different patient stays in different care units, an individual chart setup being associated with an individual care unit and with an individual viewable time frame corresponding to a patient stay in the individual care unit as well as with a set of patient medical data acquired during the patient stay in care unit; acquiring and storing medical data of a patient in the store; enabling a user to enter input data and commands via a displayed image screen; and generating in response to a user command selecting a particular care unit from a plurality of different care units, an image screen presenting an individual chart setup associated with the selected particular care unit and for presenting a set of patient medical data acquired during the patient stay in the selected particular care unit.

## Description

### Cross Reference to Related Application

This application claims the benefit of a provisional U.S. application, U. S. Serial No. 60/374,902, filed April 23, 2002 by A. M. Manetta et al.

### Field of the Invention

The present invention generally relates to a system and a method for processing and displaying of medical information, and more particularly, to processing and displaying of patient data. In one exemplary aspect, the present invention enables a user of a charting system to navigate through patient chart data in various care units during a patient's stay, in a quick and efficient manner.

### Background of the Invention

In today's medical environment, various patient data is generated during a patient's stay in a hospital. The patient data is either stored electronically or written down on paper, depending on types of data and level of automation for a particular hospital. The type of data may include parameter settings for a piece of medical equipment used to treat a patient or parameter values obtained relating to physiology of a patient.

For example, various types of medical equipment are used to monitor or administer care to patients in different hospital departments. In a critical care unit, a ventilator is frequently used to ventilate a patient's lungs with breathing gas when the patient's ability to breathe on his or her own is impaired. In order to properly administer a ventilator, a caregiver must first set up various settings for the ventilator. Examples of commonly required settings to control a ventilator include: Peak Inspiratory Pressure (PIP) setting for limiting the peak pressure during inspiration of air; and Positive End Expiratory Pressure (PEEP) setting for limiting the peak pressure at the end of expiration of air. Many other ventilator settings may also be controlled, depending on the capability of the particular ventilator.

Likewise medical equipment may also be equipped with various physiological sensors so that the condition of a patient may be monitored.

For example, commonly monitored parameters for a ventilator include Mean Airway Pressure (MAP) for indicating the mean pressure measured within the airway during the breathing cycle, and Tidal Volume Inspired (TVi) for measuring volume of gas inhaled by a patient during a normal breath. Of course, other different patient parameters may be monitored by other types of medical devices.
In addition, hospitals also have laboratories to analyze, for example, blood of a patient. The results of the blood tests may be printed out by a lab technician and given to a caregiver or stored electronically on a computer to be accessed by the caregiver. The caregiver can then analyze the results and choose a correct course of treatment for the patient.

The various exemplary patient data for a patient during his or her stay is now frequently stored electronically and often in a networked environment.

A care provider may then access the data using, for example, web browser: software through a network. This allows a caregiver to access the data throughout the hospital or even remotely through Internet.

### Summary of the Invention

The present inventors recognize that patient data for a patient's length of stay may consist of a large amount of data. If all of the data is sent to a web browser at once, the user may wait an unacceptable amount of time for the data to be down loaded and displayed.

The conventional and typical way of solving this problem is to partition data into fixed lengths of time (e.g., one day worth of data). The present inventors, however, recognize several problems with this solution.

First, patient data is usually not uniform over time. For example, on an electronic patient flowsheet, 5 minute, 15 minute, 1 hour, and 4 hour time intervals can be intermingled. Therefore, if the data is loaded and displayed in fixed time segments, the load time and amount of data displayed can vary tremendously from time period to time period.

Also, a patient's hospital length of stay may consist of stays in different care units in which the patient's set of data and chart setups vary. If more than one care unit is required, switching the view for a patient from one care unit to another generally takes several cumbersome steps in prior systems.

The present invention solves these problems by breaking patient data up into logical segments based not on a fixed time period, but on a predetermined amount of data. The present invention also gives a mechanism to a user to navigate through data in such a way as to make the segments appear seamless. Further, displaying data for any care unit during a patient's stay is easy and seamless through different navigation tools provided. Therefore, in one exemplary aspect of the present invention, a system and a method for processing medical information are described. An attempt is made to acquire patient data in response to a user request. A determination is made as to whether the patient data relating to the user request comprises more than a predetermined amount data, such as, for example, 48 columns worth of data. If the data relating to the user request comprises more than the predetermined amount, only the predetermined amount of data is acquired. Otherwise, all available data relating to the user request is acquired.

### Brief Description of the Drawings

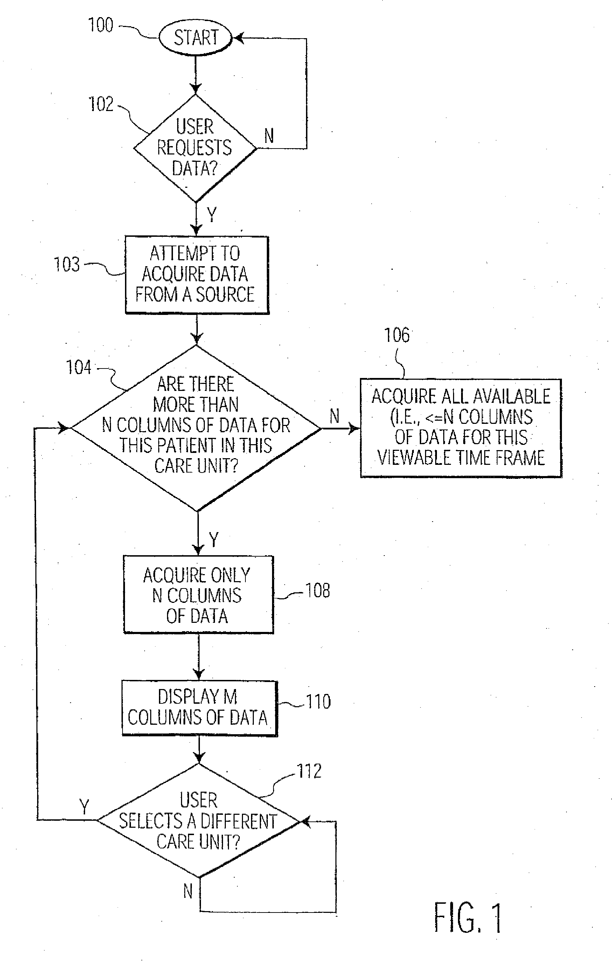

In the drawing: Fig. 1 is an exemplary process according to the present invention.
Fig. 2 shows an example of a user interface screen according to the present invention.
Fig. 3 is another example of a user interface screen having a different care unit.
Fig. 4 is a user interface screen showing viewable time frame being indicated in a calendar.
Fig. 5 is a user interface screen having applicable care units being displayed in a calendar.
Fig. 6 is an exemplary system according to the present invention.

### Detailed Description

Fig. 1 shows an exemplary method employed by a system for processing and displaying patient data according to the present invention.

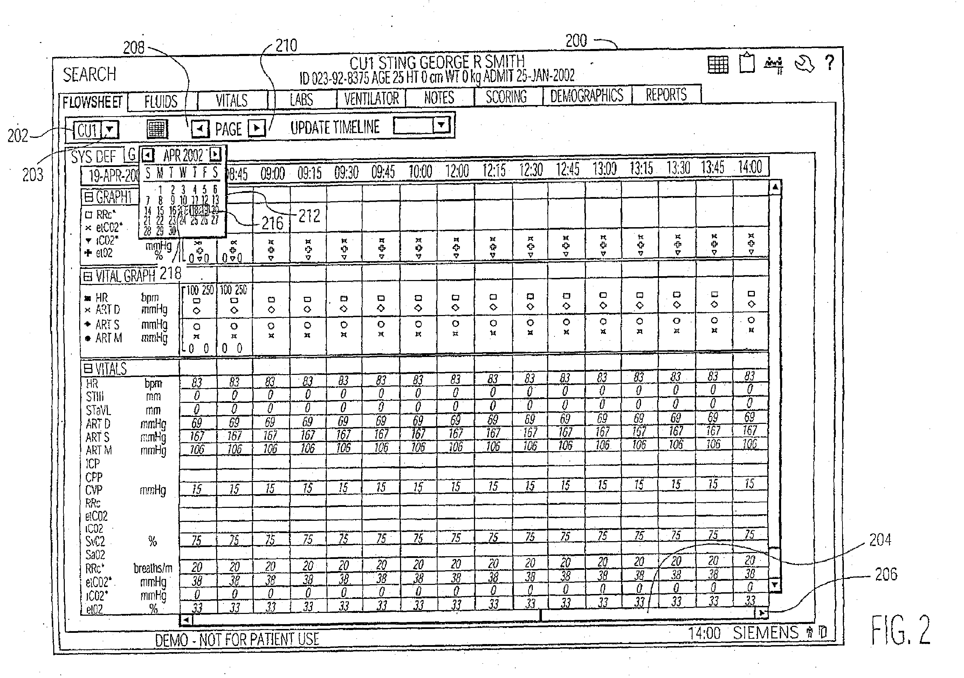

Fig. 2 shows an exemplary user interface screen which may be employed by the present system.
As shown in Fig. 1, an exemplary method of the present system will retrieve data based on a predetermined amount of data, instead of retrieving data based on a predetermined time period. At step 102 of Fig. 1, a system according to the present invention will determine if a user has made a data request. If the user has requested data, the system will then attempt at step 103 to acquire data from a predetermined source over, for example, a network. The system will determine at step 104 if the amount of data related to this user request has exceeded a predetermined amount (e.g., N columns) for! this particular patient in this particular care unit. Each user request is defined by a Viewable Time Frame (VTF), which consists up to, for example, 48 columns of data. Since granularity of data for a particular chart and/or patient varies according to how often the data has been entered and stored, a Viewable Time Frame can reflect a part of a day or several days worth of data. At step 106, if the amount of data does not, for example, comprise more than 48 columns, the system will acquire all the available columns of data related to this user request. On the other hand, at step 108, if the amount of data comprises more than 48 columns, the system will acquire only 48 columns of data for this user request.

The system will then process the acquired columns of data for display.

At step 1 10 of Fig. 1, the system displays a predetermined amount of data (e.g., M columns) on a display. The amount of data displayed may be smaller than the amount of data acquired. This allows data to be displayed in a larger font format than possible if all 48 columns of acquired data have to be displayed simultaneously. An example of displayed data is illustrated in a user interface screen 200 of Fig. 2. For example, only 16 out of 48 acquired columns of data are displayed in user interface screen 200.

Also illustrated in Fig. 2, several navigation tools are provided in user interface 200 to make navigation through patient data logical and seamless in accordance with other aspects of the present invention. For example, since in an exemplary embodiment, a Viewable Time Frame is not applied across care units, a care unit selector tool 202 of Fig. 2 is provided for navigating through the different care units during a patients length of stay. The care unit selector tool is illustrated as a pull down list of applicable care units when a down arrow 203 is selected.

This process is also illustrated in the exemplary process flow of Fig. 1.
At step 112 of Fig.1, the system will determine if a different care unit has been selected by a user via, for example, a selection tool 202 of Fig. 2. When a user selects a different care unit, Viewable Time Frame is updated.The Viewable Time Frame is updated to include up to, for example, 48! columns of data ending at the time the patient has been moved from the care unit, or ending at the current time, if the selected care unit is the current care unit. Less than 48 columns are included if 48 columns of data do not exist for the patient in the selected care unit, as shown at step 106 of Fig. 1.

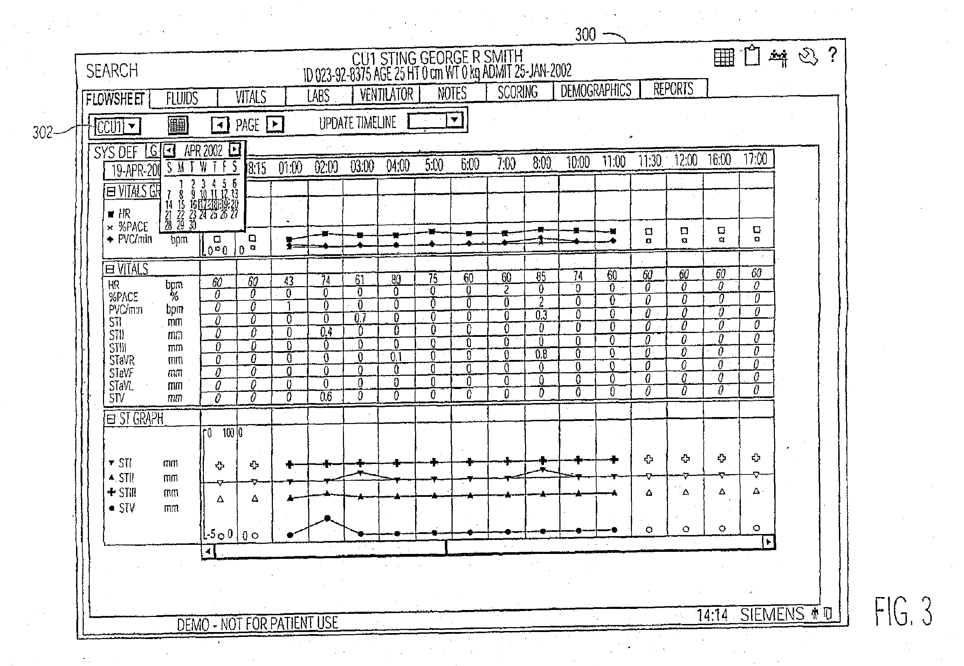

Figure 3 shows an example of a user interface screen 300 when a different care unit (e.g., CCU 1 302) is selected via the care unit selection tool for a patient. The correct setup for the selected care unit is automatically loaded when the data related to the selected care unit is loaded. Therefore, the present system provides a way for a user to quickly move from viewing data in one care unit to the next.

In another aspect of the present invention, since the amount of data displayed on a screen may be smaller than the amount of data acquired in a Viewable Time Frame, scrollbar 204 in Fig.2 is used to scroll through all the data in the currently-selected Viewable Time Frame. For example, as a user selects arrow 206 of Fig. 2, the data being displayed will be shifted so that additional data corresponding to a later time is displayed. If arrow 206 is continued to be selected, the scrolling of data continues until all 48 columns of data in the Viewable Time Frame have been displayed.

Also, as shown in Fig. 2, page foreword and backward buttons 210 and 208 are used to load the next or previous Viewable Time Frame, respectively.

For example, when the next page button 210 is selected, a new Viewable Time Frame beginning after the last column in the current Viewable Time Frame is loaded. When the previous page button 208 is selected, a new Viewable Time Frame ending just before the first column in the current Viewable Time Frame is loaded.

The next/previous Viewable Time Frame may or may not be in the same care unit. If the care unit is different, the correct setup for the new Viewable Time Frame is loaded for the corresponding care unit. A care unit select list 202 and a calendar tool 212 shown in Fig. 2 are also updated to reflect the correct history of patient's stay in the different care units. The present system thus provides a user with a way to quickly go through the patient data in chronological order.

The calendar tool 212 in Fig. 2 gives a quick access to any data in the entire patient's length of stay. Calendar tool 212 also gives a summary of a patient's hospital stay history by shading all the days that the patient has been there, as shown in Fig. 2. The day or days in the current care unit stay are more darkly shaded as shown in 216 of Fig. 2 and the day or days in previous care unit stays are more lightly shaded as shown in 218.

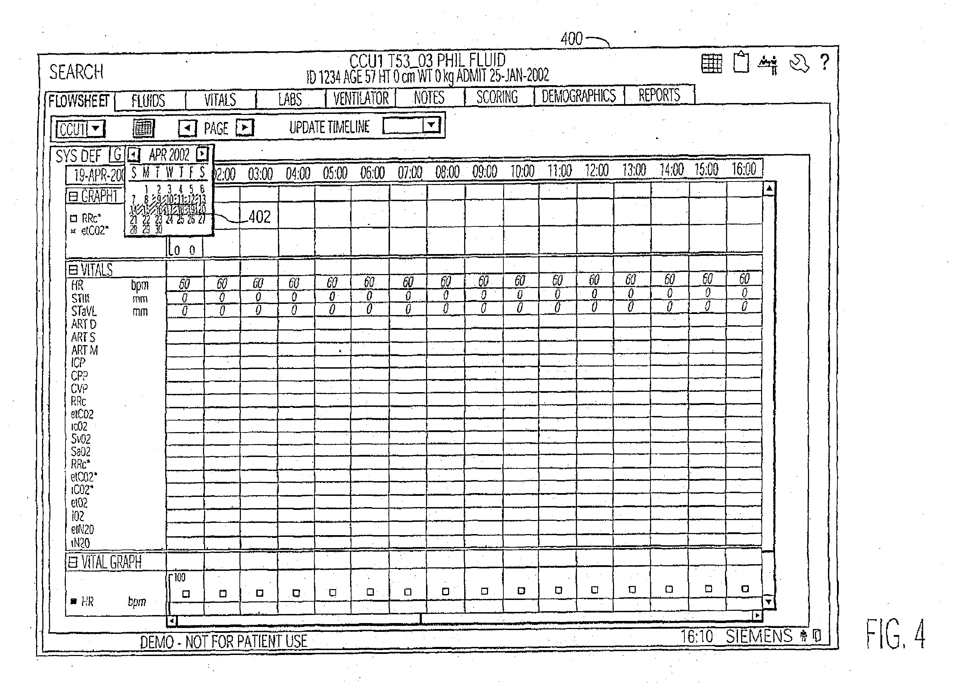

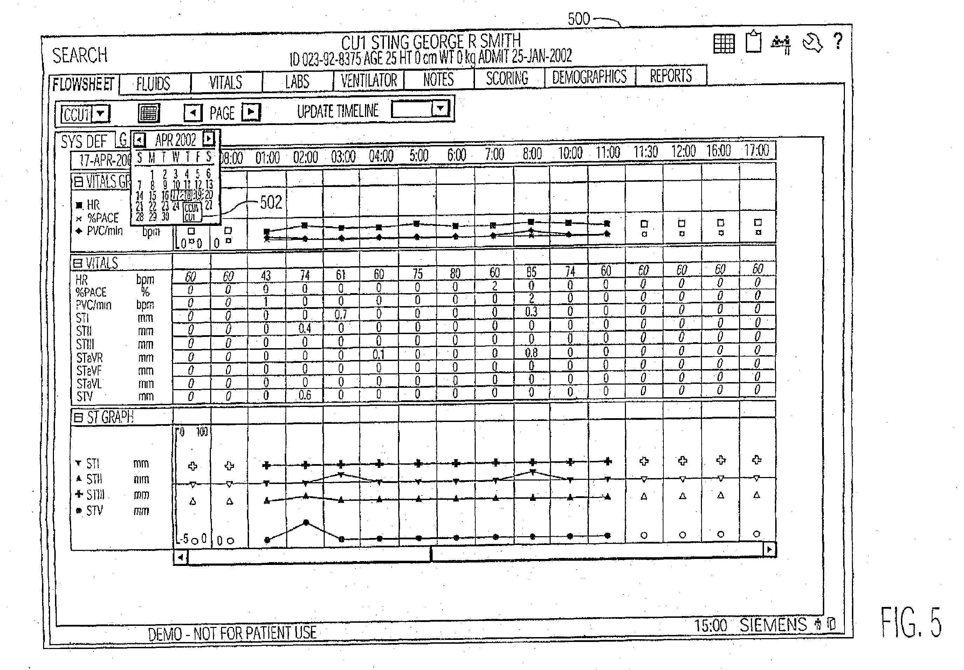

In addition, the system may also highlight the days associated with the current Viewable Time Frame by, for example, a dash box 402 of Fig. 4. Furthermore, when a user places, for example, a mouse cursor (not shown) of a user interface device over one of the shaded days in the calendar tool, the care unit or care units that the patient has been admitted to on that day, will be displayed, as shown in 502 of Fig. 5.

Thus using the calendar tool, a user may navigate directly to the data for a particular day by selecting the day on the calendar tool 212. The Viewable Time Frame is then displayed ending at midnight of the selected day. The correct care unit setup for the corresponding Viewable Time Frame is loaded and the care unit select list and the calendar are also updated automatically to reflect the correct care unit selection. Therefore, this tool provides the user the ability to jump directly to any point in the patients stay.

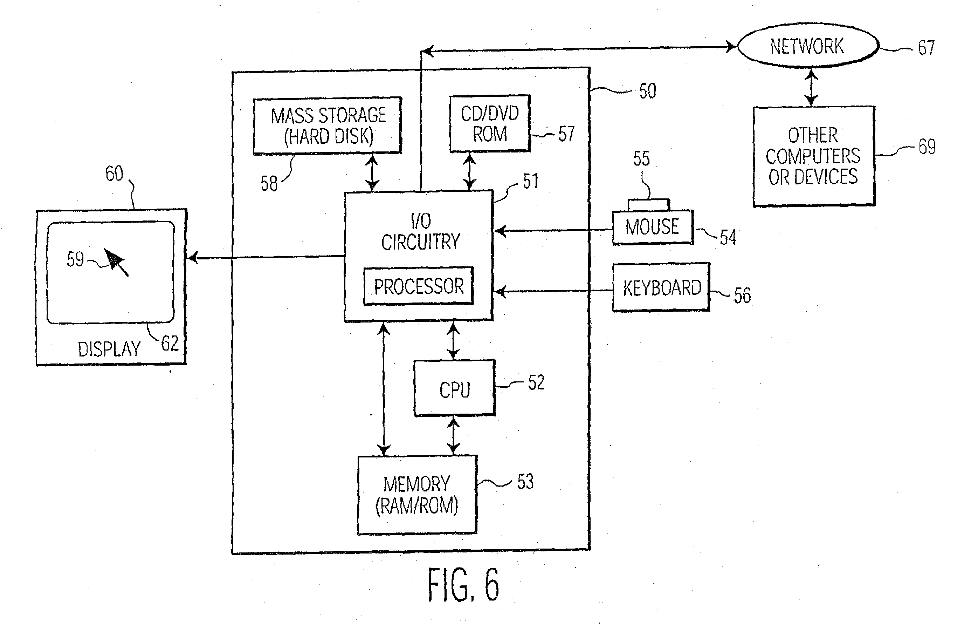

Fig. 6 describes an exemplary system in accordance with the present i invention. System 50 may comprise a general purpose computer or a specially constructed computer. A general purpose or specially constructed computer may be used with a program or programs in accordance with the I teachings herein. An example of general purpose computer may be an Intel® based personal computer, capable of running MS Windows®. An example of a specialized machine may be a patient data display system for used in a hospital.

The exemplary process of the present invention as shown in Fig. 1 may be implemented using an exemplary system illustrated in Fig. 6. System 50 of Fig. 6 comprises an input/output (I/O) section 51 which is used to communicate information in an appropriate form to and from other components of system 50. I/O section 51 may also communicate with a local area or wide are network 67, including the Internet, via for example, TCP/IP protocol. This allows system 50 to communicate with other computers or devices 69 over the network 67, via for example, a web browsing software I such as Microsoft Internet Explorers.

In addition, system 50 comprises a central processing unit (CPU) 52 coupled to I/O section 51, and a memory 53 such as RAM and/or ROM for storing computer programs and other information to be executed. An example of a computer program which may be executed by system 50 is a process illustrated in Fig. 1.

System 50 includes a display 60, such as, for example, a CRT monitor, a liquid crystal display (LCD), or others. As illustrated in Fig. 6, a user interface screen 62 is displayed on display 60. An example of a display screen 62 is shown, for example, as display screen 200 of Fig. 2 or screen 300 of Fig. 3.

System 50 further includes a cursor control 54, such as, for example, a mouse, a track ball, joystick or other device for selectively positioning a cursor 59 on a display screen 62 of the display 60. Typically, cursor control 54 includes a signal generator, such as a switch 55 which a user of the computer system may use to generate signals directing the computer to execute certain commands which have been focused or enabled by the cursor control 54. I System 50 also includes a keyboard 56 to input data and commands from a user, as is well known in the art.

Also shown in Fig. 6 is a mass storage device 58, such as a hard disk, coupled to I/O circuit 51 to provide additional storage capability for computer 50. In addition, a CD/DVD ROM 57 is further coupled to I/O circuit 50 for additional storage capacity or as another I/O device. It will be appreciated that additional devices (not shown) may be coupled to computer 50 for various purposes, as well known in the art.

The described system and method may be advantageously applied to any system, including a web-based system, needing to load large quantities of data with variable time granularity. Also, other types of predetermined I amount of data for used in the present system may be, for example, a predetermined quantity of bytes of data employed in a trend indicative display; data representing a predetermined number of columns of a flowsheet; and data corresponding to a predetermined time period of data for trend indicative display; etc. It is to be understood that the embodiments and variations shown and described herein are for illustrations only and that various modifications may be implemented by those skilled in the art without departing from the scope of the invention.

## Claims

1. A method for processing medical information, comprising the activities of:
storing information including sets of patient medical data and chart setups associated with a plurality of different patient stays in different care units, an individual chart setup being associated with an individual care unit and with an individual viewable time frame corresponding to a patient stay in the individual care unit as well as with a set of patient medical data acquired during the patient stay in care unit;
acquiring and storing medical data of a patient in the store;
enabling a user to enter input data and commands via a displayed image screen; and
generating in response to a user command selecting a particular care unit from a plurality of different care units, an image screen presenting an individual chart setup associated with the selected particular care unit and for presenting a set of patient medical data acquired during the patient stay in the selected particular care unit.

2. The method of claim 1, further comprising the activity of navigating between viewable time frames.

3. The method of claim 2, further comprising the activity of loading and displaying the acquired medical information according to a setup corresponding to a different care unit when the care unit changes during navigation.

4. The method of claim 1, further comprising the activity selecting from a calendar a particular date related to the data requested by the user, the calendar highlighting a plurality of dates a selected patient is admitted to a hospital.

5. A system for processing medical information, comprising:
a store of information including sets of patient medical data and chart setups associated with a plurality of different patient stays in different care units, an individual chart setup being associated with an individual care unit and with an individual viewable time frame corresponding to a patient stay in said individual care unit as well as with a set of patient medical data acquired during said patient stay in said care unit;
a processor for acquiring and storing medical data of a patient in said store;
a user input device enabling a user to enter input data and commends via a displayed image screen; and
a display unit for, in response to a user command selecting a particular care unit from a plurality of different care units, using said store of information to generate an image screen presenting an individual chart setup associated with the selected particular care unit and an associated individual viewable time frame corresponding to a patient stay in said selected particular care unit and for presenting a set of patient medical data acquired during said patient stay in said selected particular care unit.

6. The system of claim5, wherein said user input device includes page buttons for navigating between viewable time frames, said display unit loading and displaying the acquired medical information according to a setup corresponding to a different care unit when the care unit changes during navigation.

7. The system of claim 5, wherein said display unit uses said store of information to display an image screen presenting an individual chart setup associated with the selected particular care unit and an associated individual viewable time frame corresponding to a patient stay in said selected particular care unit and for presenting a set of patient medical data acquired during said patient stay in said selected particular care unit in response to a user command selecting a particular viewable time frame.
